(19) Europäisches Patentamt
European Patent Office
Office européen des brevets

(11) Veröffentlichungsnummer: **0 193 656 B1**

(12) **EUROPÄISCHE PATENTSCHRIFT**

(45) Veröffentlichungstag der Patentschrift: **25.04.90**

(51) Int. Cl.⁴: **A61K 7/13**

(21) Anmeldenummer: **85202032.0**

(22) Anmeldetag: **09.12.85**

(54) **Haarfärbemittel.**

(30) Priorität: **08.03.85 DE 3508265**

(43) Veröffentlichungstag der Anmeldung: **10.09.86 Patentblatt 86/37**

(45) Bekanntmachung des Hinweises auf die Patenterteilung: **25.04.90 Patentblatt 90/17**

(84) Benannte Vertragsstaaten: **CH DE FR GB IT LI**

(56) Entgegenhaltungen:
**EP-A- 0 137 524**
**CH-A- 464 448**
**DE-A- 1 949 750**

**J. HETEROCYCLIC. CHEM., Band 18, Nr. 8, Dezember 1981, Seiten 1581-1585; K. RASHEED et al.: "Thermal decompositions of dinitropyridyl and dinitrothienyl dithiocarbamates and t-butyl trithiocarbonates"**

**Die Akte enthält technische Angaben, die nach dem Eingang der Anmeldung eingereicht wurden und die nicht in dieser Patentschrift enthalten sind.**

(73) Patentinhaber: **RÜTGERSWERKE AKTIENGESELLSCHAFT, Mainzer Landstrasse 217, D-6000 Frankfurt am Main 1(DE)**

(72) Erfinder: **Orth, Winfried, Dr., Am Schachtelgraben 28, D-6733 Hassloch/Pfalz(DE)**
Erfinder: **Schrader, Karl-Heinz, Dr., Erlengrund 16, D-3454 Bevern(DE)**
Erfinder: **Fickert, Werner, Dr., Stockacher Strasse 14, D-6800 Mannheim 61(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft Haarfärbemittel auf der Basis von Oxidationsfarbstoffen in Kombination mit einschlägigen Entwicklern. Die Färbung der Haare erfolgt dabei durch Reaktion der Entwicklersubstanzen mit sogenannten Kupplersubstanzen oder Nuanceuren in alkalischem Medium in Gegenwart eines geeigneten Oxidationsmittels. Durch die im allgemeinen dabei entstehenden intensiven Farben mit sehr guten Echtheitseigenschaften und durch die große Variationsbreite der Farbtöne spielen diesen Oxidationsfarben eine bedeutende Rolle in der Haarkosmetik.

Als Kuppler- oder Nuanceurkomponenten kennt man m-Phenylendiaminderivate, Phenole, Naphthole oder Resorcinderivate. Da diese Produkte alle toxikologisch und dermatologisch nicht unbedenklich sind, versucht man, auf die unbedenklichere Pyridinaminoverbindungen überzugehen.

So kennt man die Verwendung als Kupplersubstanz von
2,3- bis 2,6-Diaminopyridin aus DE-PS 1 142 045
2,5-Diaminopyridin aus DD-PS 57 402
Bis-aminopyridinen aus EP 0 008 079 B 1
Dihydroxipyridinen aus FR-PS 1 398 193
Hydroxi- und Alkoxipyridinaminen aus FR-PS 1 397 551
und FR-PS 1 398 193
oder
Pyridylaminobenzolen und Bispyridylaminen aus FR-PS 1 401 469. Aminopyridinverbindungen aber sind oxidationsempfindlich gegen Luftsauerstoff. Um Verluste, die bereits bei der Lagerung der Präparate sowie bei Gebrauch entstehen, auszugleichen, werden diese Verbindungen in größeren Mengen eindosiert als sie für den Färbvorgang an sich notwendig wären. Außerdem werden diese Aminoverbindungen als Salze eingesetzt, um somit eine Stabilisierung während der Lagerung zu erzielen. Dadurch aber handelt man sich andere Nachteile ein: Aufgrund der durch die Salzbildung bedingten höheren Anzahl an Ionen wird die Brillanz der Farbtöne gemindert. Außerdem sind diese Salze in höheren Konzentrationen nicht mit allen Tensiden kombinierbar, so daß nicht alle wünschenswerten galenischen Formen der Haarfärbemittel herstellbar sind.

Es bestand daher die Aufgabe, Haarfärbemittel auf Basis von Oxidationsfarbstoffen zu entwickeln, die intensive Farben mit sehr guten Echtheitseigenschaften in einer großen Variationsbreite der Farbtöne ergeben und deren Kuppler- oder Nuanceurkomponenten toxikologisch und dermatologisch unbedenklich und gegen Luftsauerstoff weitgehend beständig sind, so daß sie auch in geringeren Mengen in neutraler oder Salzform eingesetzt werden können.

Die Lösung der Aufgabe erfolgt durch Bereitstellung der Mittel gemäß Anspruch 1.

Es wurde gefunden, daß Dinitropyridinderivate der allgemeinen Formel I

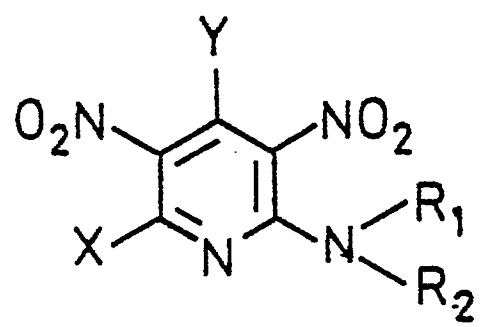

(I)

in Kombination mit einschlägigen Entwicklerkomponenten und Oxidationsmitteln ideale Kuppler- oder Nuanceurkomponenten darstellen, die die oben genannten Anforderungen erfüllen.

Es ist allgemein bekannt, daß die Nitrogruppe die Oxidationsbeständigkeit von Pyridinderivaten steigert. Diese Kenntnis hat denn auch bislang den Einsatz von Nitropyridinderivaten in Färbemitteln auf Basis von Oxidationsfarbstoffen verhindert, denn die dort eingesetzten Produkte müssen ja mit einem gängigen Oxidationsmittel leicht reagieren. Daher sind bestimmte Nitropyridinderivate zwar aus DE-PS 1 949 750 als selbstaufziehende Haarfarben sowie aus CH-A 464 448 als Kupplerkomponente für Azofarbstoffe zum Färben von Haaren bekannt, nicht aber ihre Verwendung in Färbemitteln auf Basis von Oxidationsfarben. Überraschenderweise sind sogar die Dinitropyridinderivate gemäß der Erfindung zwar gegen Luftsauerstoff ausreichend stabil, daß sie auch in ihrer nichtionogenen Form eingesetzt werden können, aber trotzdem so reaktiv, daß sie mit gängigen Oxidationsmitteln in der an sich bekannten Weise im Sinne der Färbung reagieren.

Es wurde weiterhin gefunden, daß die Dinitropyridinderivate gemäß der Erfindung sowohl toxikologisch als auch dermatologisch als unbedenklich angesehen werden können. Dies ist insofern überraschend, als aus der Aromatenchemie bekannt ist, daß die Nitroprodukte allgemein eine höhere Toxizität zeigen als die nicht nitrierten Aromaten.

Weitere Vorteile der erfindungsgemäßen Haarfärbemittel sind äußerst brillante Farbtöne, die sich bei Verwendung der als Kupplersubstanzen bekannten Aminopyridinen nicht erzielen lassen, sowie eine stärkere Farbgebung trotz niedriger Einsatzmengen. Dieser letzte Effekt ist teils bedingt durch die farbgebende Wirkung der Nitrogruppen und teils durch Oxidationsstabilität. Dadurch wird es ermöglicht, mit etwa der halben Menge der erfindungsgemäßen Dinitropyridinderivate anstelle der bislang verwendeten Aminopyridinderivate gleich tiefe, aber brillantere Farbtöne zu erzielen.

Durch Variation der Substituenten am Pyridinring und an der Aminogruppe der erfindungsgemäß eingesetzten Dinitropyridinverbindungen lassen sich verschiedene Farbvarianten erzielen. Es ist somit mit diesen Mitteln möglich, durch Abmischung verschiedener Nuanceurkomponenten viele Farbvarianten mit einem Oxidationsfarbensystem einzustellen. Die erfindungsgemäßen Haarfärbemittel stellen somit eine Bereicherung auf dem Gebiet der Haarkosmetik dar.

Erfindungsgemäß in Oxidationsfarbstoffsystemen einzusetzende Kuppler- und Nuanceurkomponenten sind theoretisch alle Pyridinderivate, die je eine Nitrogruppe in 3- und 5-Stellung und mindestens eine substituierte oder unsubstituierte Aminogruppe in 2-Stellung am Pyridinring enthalten, wobei die Position 6 entweder unsubstituiert oder durch eine Alkoxi-, Hydroxialkyl-, Aminoalkyl- oder eine substituierte oder unsubstituierte Aminogruppe substituiert und die Position 4 entweder unsubstituiert oder durch eine kurzkettige Alkyl- oder eine unsubstituierte oder substituierte Phenylgruppe substituiert ist. Verkaufsfähige Produkte sollen jedoch in Wasser ggf. als Salz oder im Gemisch mit einem Lösungsvermittler löslich und stabil sein, so daß von daher gewisse Einschränkungen bezüglich der Substituenten vorgegeben sind. In der Praxis werden daher die Dinitropyridinderivate bevorzugt, die der allgemeinen Formel (I) entsprechen.

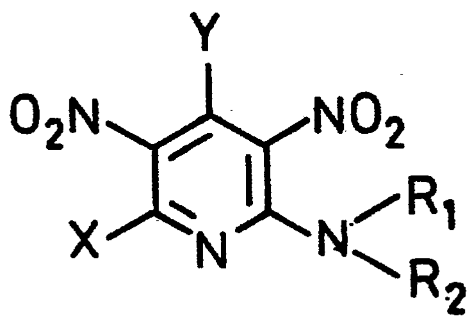 . (I)

Die Substituenten haben die folgenden Bedeutungen: X kann Wasserstoff, eine Aminoalkyl-, Hydroxialkyl-, oder Alkoxigruppe mit 1 - 4 C-Atomen oder eine Aminogruppe sein. Beispiele für derartige Substituenten sind die folgenden Gruppen: Aminomethyl-, Aminoäthyl-, Aminopropyl-, Amino-i-propyl-, Aminobutyl-, Hydroximethyl-, Hydroxiäthyl-, Hydroxipropyl-, Hydroxibutyl-, Methoxi-, Äthoxi- oder Propoxi-. Die Aminogruppe entspricht wie die Aminogruppe in 2-Stellung der allgemeinen Formel:

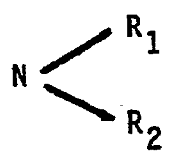

Diese Aminogruppen in 2- und 6-Stellung können gleich oder verschieden sein. Y kann Wasserstoff, eine Methyl-, Äthyl-, Propyl-, i-Propyl-, Phenyl-, Hydroxiphenyl- oder eine Aminophenylgruppe sein.

Der Substituent $-NR_1R_2$ kann eine unsubstituierte oder substituierte Aminogruppe sein, wobei $R_1$ und $R_2$ gleich oder verschieden sein können und Wasserstoff, einen Alkylrest mit 1 - 4 C-Atomen, einen unsubstituierten oder an beliebiger Stelle mit einer oder mehreren Amino-, Methylamino-, Dimethylamino-, Hydroxi-, Alkyl- oder Alkoxigruppe mit 1 - 2 C-Atomen substituierten Phenylrest, einen Cycloalkylrest mit bis zu 7 C-Atomen oder einen unsubstituierten oder an beliebiger Stelle mit einer Methyl-, Äthyl-, oder Propylgruppe gruppe substituierten heterocyclischen Rest darstellen.

Beispiele für diese Aminogruppe sind die Methyl-, Äthyl-, Propyl-, i-Propyl, Butyl-, i-Butyl, tert. Butyl-, Dimethyl-, Diäthyl-, Methyl-äthyl-, Dipropyl-, Di-i-propyl-, Methylpropyl-, Cyclohexyl-, Phenyl-, Aminophenyl-, Diaminophenyl-, Methylaminophenyl-, Dimethylaminophenyl-, Hydroxiphenyl-, Dihydroxiphenyl-, Tolyl-, Xylyl-, Äthylphenyl-, Methoxiphenyl-, Äthoxiphenyl- oder Dimethoxiphenylaminogruppe sowie ein Pyrrol-, Methylpyrrol-, Äthylpyrrol-, Propylpyrrol-, Pyridin-, Methylpyridin-, Äthylpyridin-, Dimethylpyridin-, Propylpyridin-, Piperidin-, Methylpiperidin-, Äthylpiperidin-, Propylpiperidin-, Pyrimidin-, Methylpyrimidin-, Äthylpyrimidin-, Propylpyrimidin-, Piperazin-, Methylpiperazin, Äthylpiperazin-, Propylpiperazin-, Morpholin-, Methylmorpholin-, Äthylmorpholin- oder Propylmorpholinrest.

Die Substituenten $R_1$ und $R_2$ können aber auch einen Alkenylrest der allgemeinen Formel

-R-Z

darstellen, wobei R eine Alkylkette mit 1 - 6 C-Atomen oder eine Phenylgruppe und Z eine an beliebiger Stelle stehende Hydroxi- und/oder Alkoxigruppe mit 1 - 3 C-Atomen oder eine Aminogruppe der allgemeinen Formel

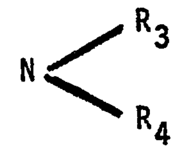 bedeuten.

$R_3$ und $R_4$ sind gleich oder verschieden und bedeuten Wasserstoff, einen unsubstituierten cder an beliebiger Stelle hydroxi- oder aminosubstituierten Alkyl-, Aralkyl-, Cycloalkylrest mit bis zu 7 C-Atomen einen unsubstituierten oder an beliebiger Stelle mit einer oder mehreren Amino-, Methylamino-, Dimethylamino-, Hydroxi-, Alkyl- oder Alkoxigruppe mit 1 - 2 C-Atomen substituierten Phenylrest oder einen unsubstituierten oder an beliebiger Stelle methyl-, äthyl- oder propylsubstituierten Pyrrol-, Pyridin-, Piperidin-, Pyrimidin- Piperazin- oder Morpholinrest. Beispiele für derartige Aminogruppen sind die Hydroximethyl-, Hydroxiäthyl-, Hydroxipropyl-, Hydroisopropyl-, Hydroxibutyl-, Hydroxipentyl-, Hydroxihexyl-, Methoximethyl-, Äthoximethyl-, Propoxmethyl-, Isopropoximethyl-, Hydroximethoximethyl-, Hydroxiäthoximethyl-, Methoxiäthyl-, Äthoxiäthyl-, Aminomethyl-, Aminoäthyl-, Aminopropyl-, Aminobutyl-, Aminopentyl-, Aminohexyl-, Aminocyclohexyl-, Methylaminomethyl-, Methylaminoäthyl-, Methylaminopropyl-, Dimethylaminomethyl-, Dimethylaminoäthyl-, Dimethylaminopropyl-, Äthylaminomethyl-, Propylaminomethyl-, Diäthylaminomethyl-, Äthylaminoäthyl-, Diäthylaminoäthyl-, Äthylaminopropyl-, Diäthylaminopropyl-, Bis-aminomethyl-, Bis-aminoäthyl-, Bisaminopropyl-, Bis-aminobutyl-, Bis-aminopentyl-, Bis-aminohexyl-, Hydroxiphenyl-, Methoxiphenyl-, Tolyl-, Dimethoxiphenyl-, Aminophenyl-, Phenyl-, Diaminophenyl-, Pyrrolmethyl-, Pyrroläthyl-, Methylpyrrolmethyl-, Methylpyrroläthyl-, Pyridinmethyl-, Pyridinäthyl-, Pyridinpropyl-, Methylpyridinmethyl-, Methylpyridinäthyl-, Dimethylpyridinmethyl-, Dimethylpyridinäthyl-, Äthylpyridinmethyl-, Äthylpyridinäthyl-, Piperidinmethyl-, Piperdinäthyl-, Methylpiperidinmethyl-, Äthylpiperidinäthyl-, Piperazinmethyl-, Piperazinäthyl-, Methylpiperazinmethyl-, Methylpiperazinäthyl-, Äthylpiperazinmethyl-, Äthylpiperazinäthyl-, Pyrimidinmethyl-, Pyrimidinäthyl-, Pyrimidinpropyl-, Methylpyrimidinmethyl-, Morpholinmethyl-, Methylmorpholinmethyl-, Äthylmorpholinmethyl-, Morpholinäthyl-, Methylmorpholinäthyl-, Äthylmorpholinäthyl-, Propylpyrrolmethyl-, Propylpyridinmethyl-, Propylpiperinmethyl-, Propylpyrimidinmethyl-, Propylpiperazinmethyl- oder Propylmorpholinmethylamin.
Die erfindungsgemäßen Nuanceurkomponenten können jeweils alleine oder zur Einstellung gewünschter Farbnuancen in Abmischung miteinander oder mit anderen an sich bekannten Nuanceur- oder Kupplerkomponenten eingesetzt werden.

Als Beispiel für in den erfindungsgemäßen Haarfärbemitteln einzusetzende Entwicklerkomponenten sind primäre aromatische Amine mit einer weiteren in p-Stellung befindlichen funktionellen Gruppe wie
p-Phenylendiamin, Alkylamino-p-phenylendiamine
p-Toluylendiamin,
p-Aminophenol,
N-Methyl-p-phenylendiamin
N,N-Dimethyl-p-phenylen diamin,
N,N-Diäthyl-2-methyl-p-phenylendiamin,
N-Äthyl-N-hydrox äthyl-p-phenylendiamin,
Chlor-p-phenylendiamin,
N,N-Bis-hydroxiäthylamino-p-phenylendiamin,
Methoxy-p-phenylendiam n,
2,6-Dichlor-p-phenylendiamin,
2-Chlor-6-brom -p-phenylendiamin,
2-Chlor-6-methyl-p-phenylendiamin,
6-Methoxy 3-methyl-p-phenylendiamin,
andere Verbindungen der genannten Art, die weiterhin eine oder mehrere funktionelle Gruppen wie OH-Gruppen, $NH_2$-Gruppen, NHR-Gruppen, $NR_2$-Gruppen, wobei R einen Alkyl- oder Hydroxialkylrest mit 1 - 4 Kohlenstoffatomen darstellt, ferner
heterocyclische Hydrazonderivate wie
1-Methylpyrrolidon-(2)-hydrazon,
4-Aminopyrazonlonderivate wie
4-Amino-1-phenyl-3-carbamoylpyrazolon-5,
N-Butyl-N-sulfobut l-p-phenylendiamin,
Tetraaminopyrimidine wie
2,4,5,6-Tetraaminopyrimidin,
4,5-Diamino-2,6-bismethylaminopyrimidin,

2,5-Diamino-4-diäthylamino-6-methylaminop yrimidin,
2,4,5-Triamino-6-dimethylaminopyrimidin,
2,4,5-Triami o-6-piperidino-pyrimidin,
2,4,5-Triamino-6-anilino- pyrimidin,
2,4,5-Triamino-6-morpholinopyrimidin,
2,4,5-Triamino 6-ß-hydroxy-äthylaminopyrimidin
aber auch Pyridinderivate wie z.B. 2,5-Diaminopyridin oder 2,5-Diamino-4-methylpyridin anzuführen.

Die oxidative Kupplung, d.h. die Entwicklung der Färbung könnte grundsätzlich, wie bei anderen Oxidationshaarfarbstoffen auch durch Luftsauerstoff erfolgen. Jedoch ist für die praktische Anwendung die Reaktionsgeschwindigkeit zu gering und die Farbentwicklung auf dem Haar zu langsam. Daher werden bevorzugt chemische Oxidationsmittel eingesetzt. Als solche kommen insbesondere Wasserstoffperoxid oder dessen Anlagerungsprodukte an Harnstoff, Melamin und Natriumborat sowie Gemische aus derartigen Wasserstoffperoxidanlagerungsverbindungen mit Kaliumperoxidisulfat in Betracht.

Die erfindungsgemäßen Haarfärbemittel, die die Nuanceur- und Entwicklerkomponente enthalten, werden für den Einsatz in entsprechende kosmetische Zubereitungen wie Cremes, Emulsionen, Gele oder auch einfach Lösungen eingearbeitet. Dazu ist es mitunter notwendig, die Lösungen bis zu 100°C zu erhitzen, um die Komponenten in Lösung zu bringen, ggf. unter Mithilfe eines Lösungsvermittlers. Dabei liegen in gebrauchsfähigen Produkten die Konzentrationen an Nuanceurkomponenten zwischen 0,01 - 2 Gewichtsprozent und an Entwicklerkomponente zwischen 0,1 - 5 Gewichtsprozent. Zur Herstellung der kosmetischen Zubereitungen werden die Komponenten mit den für derartige Zubereitungen üblichen weiteren Bestandteilen gemischt. Als solche zusätzlichen Bestandteile sind zum Beispiel Ammoniumhydroxid, Netz- oder Emulgiermittel vom anionischen oder nichtionogenen Typ wie Alkylbenzolsulfonate, Fettalkoholsulfonate, Fettalkholäthersulfate, Aminoxide, Alkylsulfonate, Fettsäurealkanolamide, Alkylphenoloxathylate und Anlagerungsprodukte von Äthylenoxid an Fettalkohole, Reduktionsmitteln, wie Natriumsulfit, Natriumdithionit, Thioglykolsäure oder Ascorbinsäure, Verdickungsmittel wie Methylcellulose, höhere Fettalkohole, Fettsäuren, ferner Parfümöle und Haarpflegemittel wie Pantothensäure und Cholesterin zu nennen.

Kurz vor der Anwendung werden diese Haarfärbemittel mit einer Lösung eines der genannten Oxidationsmittel wie üblich gemischt und die so erhaltene Mischung auf das Haar aufgetragen. Die Anwendungstemperaturen bewegen sich dabei im Bereich von 30 bis 40°C. Nach einer Einwirkungsdauer von ca. 30 Minuten wird das Haarfärbemittel vom zu färbenden Haar durch Spülen entfernt. Hernach wird das Haar mit einem milden Schampoo nachgewaschen und getrocknet.

Beispiele

mit Haarfärbemitteln folgender Grundzusammensetzung werden Färbeversuche durchgeführt:

2 Gew.-% 30%ige Fettsäureaminoxidlösung
0,5 Gew.-% Natriumdithionit
10 Gew.-% 25%iges Ammoniumhydroxid
0,5 Gew.-% erfindungsgemäßer Nuanceurkomponente
1 Gew.-% p-Toluylendiaminsulfat
86 Gew.-% Wasser

100 ml der Haarfärbemittel werden mit 10 ml Wasserstoffperoxidlösung (6 %) gemischt. In diese Mischungen werden Haarsträhnen aus Naturhaar eingetaucht und die Farblösung läßt man 30 Minuten bei 35°C einwirken. Anschließend werden die Strähnen gut ausgespült, getrocknet und hinsichtlich ihrer Färbung beurteilt.

Verwendete erfindungsgemäße Nuanceurkomponente

Beispiel 1

2-Amino-6-methoxy-3,5-dinitropyridin
Farbeffekt: Braunfärbung mit leichtem Grünstich

Beispiel 2

2-Dimethylamino-3,5-dinitropyridin
Farbeffekt: Blauschwarz

Beispiel 3

2,6-bis-(2-Hydroxyäthylamin)-3,5-dinitropyridin
Farbeffekt: Haselnußartige Braunfärbung

Beispiel 4

6-Methoxy-3,5-dinitro-2-propylaminopyridin
Farbeffekt: Haselnußartige Braunfärbung

Beispiel 5

2,6-bis (N,N-dimethylamino)-3,5-dinitropyridin
Farbeffekt: Mittelblond

**Patentansprüche**

1. Mittel zum Färben von Haaren auf der Basis von Oxidationsfarbstoffen in Kombination mit einschlägigen Entwicklern, gekennzeichnet durch einen Gehalt an einem oder mehreren Dinitropyridinderivaten der allgemeinen Formel (I)

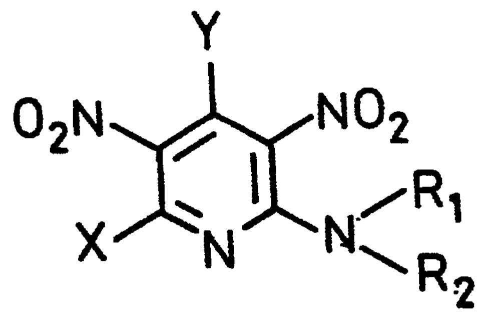

(I)

wobei $R_1$ und $R_2$ gleich oder verschieden sind und Wasserstoff, einen Alkylrest mit 1 - 4 C-Atomen, einen unsubstituierten oder an beliebiger Stelle mit einer oder mehreren Amino-, Methylamino-, Dimethylamino-, Hydroxy-, Alkyl- oder Alkoxigruppen mit 1 - 2 C-Atomen substituierten Phenylrest, einen Cycloalkylkylrest mit bis zu 7 C-Atomen oder einen unsubstituierten oder an beliebiger Stelle methyl-, äthyl- oder propylsubstituierte Pyrrol-, Pyridin-, Piperidin-, Pyrimidin-, Piperazin- oder Morpholinrest oder einen Alkenylrest der allgemeinen Formel II

-R-Z (II)

darstellen, wobei R eine Alkylkette von 1 - 6 C-Atomen oder eine Phenylgruppe und Z eine an beliebiger Stelle stehende Hydroxi- und/oder eine Alkoxigruppe mit 1 - 3 C-Atomen oder eine Aminogruppe der allgemeinen Formel III

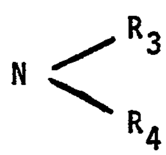

(III)

darstellt, in der $R_3$ und $R_4$ gleich oder verschieden sind und Wasserstoff einen unsubstituierten oder an beliebiger Stelle hydroxi- oder aminsubstituierten Alkyl-, Aralkyl-, Cycloalkylrest mit bis zu 7 C-Atomen, einen unsubstituierten oder an beliebiger Stelle mit einer oder mehreren Amino-, Methylamino-, Dimethylamino-, Hydroxi-, Alkyl- oder Alkoxigruppen mit 1 - 2 C-Atomen substituierten Phenylrest oder einen unsubstituierten oder an beliebiger Stelle methyl-, äthyl-, oder propylsubstituierten Pyrrol-, Pyridin-, Piperidin-, Pyrimidin-, Piperazin- oder Morpholinrest bedeuten und X entweder Wasserstoff, eine Hydroxi- oder Aminoalkyl- oder Alkoxigruppe mit 1 - 4 C-Atomen oder eine Aminogruppe der allgemeinen Formel I und Y Wasserstoff, eine Alkylgruppe mit 1 - 3 C-Atomen oder eine unsubstituierte oder hydroxi- oder aminsubstituierte Phenylgruppe darstellen.

**Claims**

1. An agent for dyeing hair on the basis of oxidation dyes in combination with appropriate developers, characterized by a content of one or more dinitropyridine derivatives of the general formula (I):

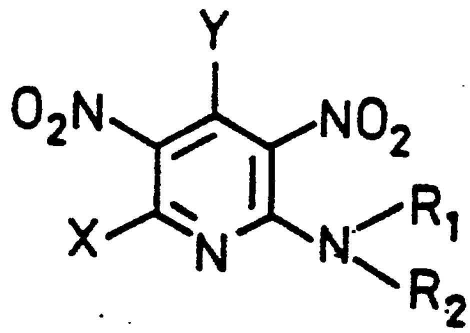

(I)

in which $R_1$ and $R_2$ are the same or different and represent hydrogen, an alkyl radical with from 1 to 4 carbon atoms, an unsubstituted phenyl radical or a phenyl radical substituted at any position with one or more amino, methyl amino, dimethyl amino, hydroxy, alkyl or alkoxy groups with 1 or 2 carbon atoms, a cycloalkyl radical with up to 7 carbon atoms or an unsubstituted pyrrol, pyridine, piperidine, pyrimidine, piperazine or morpholine radical or a pyrrol, pyridine, piperidine, pyrimidine, piperazine or morpholine radical substituted at any position with methyl, ethyl or propyl, or an alkenyl radical of the general formula (II):

$$-R-Z \quad . \qquad (II)$$

in which R represents an alkyl chain of from 1 to 6 carbon atoms or a phenyl group and Z represents an hydroxy and/or an alkoxy group standing at any position with from 1 to 3 carbon atoms or an amino group of the general formula (III):

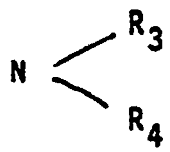

(III)

in which $R_3$ and $R_4$ are the same or different and represent hydrogen, an unsubstituted alkyl, aralkyl, cycloalkyl radical or an alkyl, aralkyl, cycloalkyl radical substituted with hydroxy or amine with up to 7 carbon atoms, an unsubstituted phenyl radical or a phenyl radical substituted at any position with one or more amino, methyl amino, dimethyl amino, hydroxy, alkyl or alkoxy groups with 1 or 2 carbon atoms, or an unsubstituted pyrrol, pyridine, piperidine, pyrimidine, piperazine or morpholine radical or a pyrrol, pyridine, piperidine, pyrimidine, piperazine or morpholine radical substituted at any position with methyl, ethyl or propyl, and X represents either hydrogen, an hydroxy or amino alkyl or alkoxy group with from 1 to 4 carbon atoms or an amino group of the general formula I, and Y represents hydrogen, an alkyl group with from 1 to 3 carbon atoms or an unsubstituted phenyl group or an hydroxy- or amine-substituted phenyl group.

## Revendications

1. Agent pour la coloration des cheveux à base de matières colorantes d'oxydation en combinaison avec des agents de développement appropriés, caractérisé par une teneur en un ou plusieurs dérivés de dinitropyridine de formule générale (I)

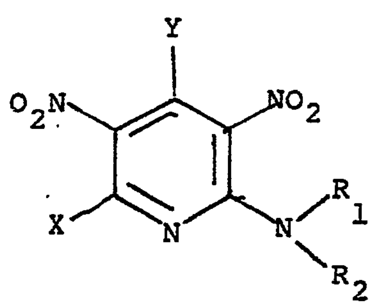

(I)

dans laquelle $R_1$ et $R_2$ sont identiques ou différents et représentent l'hydrogène, un radical alkyle comportant 1-4 atomes de C, un radical phényle non substitué ou substitué en une position quelconque par

un ou plusieurs groupes amino, méthylamino, diméthylamino, hydroxy, alkyle ou alcoxy comportant 1-2 atomes de C, ou représentent un radical cycloalkyle comportant jusqu'à 7 atomes de C ou un radical pyrrole, pyridine, pipéridine, pyrimidine, pipérazine ou morpholine non substitué ou substitué en une position quelconque par un méthyle, un éthyle ou un propyle, ou représentent un radical alcényle de formule générale II

$$-R-Z \qquad \text{(II)}$$

dans laquelle R représente une chaîne alkyle comportant 1-6 atomes de C ou un groupe phényle et Z représente un groupe hydroxy et/ou un groupe alcoxy comportant 1-3 atomes de C, en n'importe quelle position ou un groupe amino de formule générale III

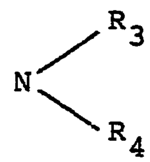

(III)

dans laquelle $R_3$ et $R_4$ sont identiques ou différents et représentent l'hydrogène, un radical alkyle, aralkyle, cycloalkyle comportant jusqu'à 7 atomes de C, non substitué ou substitué en une position quelconque par un hydroxy ou un amino, ou représentent un radical phényle non substitué ou substitué en une position quelconque par un ou plusieurs groupes amino, méthylamino, diméthylamino, hydroxy, alkyle ou alcoxy comportant 1-2 atomes de C ou représentent un radical pyrrole, pyridine, pipéridine, pyrimidine, pipérazine ou morpholine non substitué ou substitué en une position quelconque par un méthyle, un éthyle ou un propyle et X représente soit l'hydrogène, un groupe hydroxy ou aminoalkyle ou alcoxy comportant 1-4 atomes de C, soit un groupe amino de formule générale I et Y représente l'hydrogène, un groupe alkyle comportant 1-3 atomes de C ou un groupe phényle non substitué ou substitué par un hydroxy ou un amino.